# EUROPEAN PATENT SPECIFICATION

(11) **EP 3 502 683 B1**
(45) Date of publication and mention of the grant of the patent: **02.02.2022**
(21) Application number: 18214194.5
(22) Date of filing: 19.12.2018
(51) Int. Cl.: G01N 30/88

(54) **METHOD FOR CONTROLLING IMPURITY OF CYCLOSPORIN IN AN EYE GEL**
VERFAHREN ZUR KONTROLLE DER VERUNREINIGUNG VON CYCLOSPORIN IN EINEM AUGENGEL
PROCÉDÉ DE RÉGULATION DE L'IMPURETÉ D'UN GEL OCULAIRE À LA CYCLOSPORINE A

(30) Priority: 21.12.2017 CN 201711391728
(43) Date of publication of application: 26.06.2019
(73) Proprietor: Zhaoke (Guangzhou) Ophthalmology Pharmaceutical Ltd., Guangzhou, Guangdong Province (CN)
(72) Inventor: Li, Gang, Hefei City, Anhui 230088 (CN); Cao, Kailei, Hefei City, Anhui 230088 (CN); Li, Xiaoyi, Shatin (HK); Dai, Xiangrong, Hefei City, Anhui 230088 (CN); Yin, Lei, Hefei City, Anhui 230088 (CN); Ling, Juan, Hefei City, Anhui 230088 (CN)
(74) Representative: Isern Patentes y Marcas S.L.

(56) References cited:
- BONIFACIO F N ET AL: "Development and validation of HPLC method for the determination of Cyclosporin A and its impurities in Neoral(R) capsules and its generic versions", JOURNAL OF PHARMACEUTICAL AND BIOMEDICAL ANALYSIS, vol. 49, no. 2, 20 February 2009 (2009-02-20), pages 540-546, XP027282464, NEW YORK, NY, US ISSN: 0731-7085 [retrieved on 2008-11-27]
- HUSEK A ED - CHANKVETADZE B ET AL: "High-performance liquid chromatographic analysis of cyclosporin A and its oral solutions", JOURNAL OF CHROMATOGRAPHY A, vol. 759, no. 1, 24 January 1997 (1997-01-24), pages 217-224, XP004049990, AMSTERDAM, NL ISSN: 0021-9673, DOI: 10.1016/S0021-9673(96)00770-4
- JAIN DEEPTI ET AL: "Forced degradation and impurity profiling: Recent trends in analytical perspectives", JOURNAL OF PHARMACEUTICAL AND BIOMEDICAL ANALYSIS, vol. 86, 31 July 2013 (2013-07-31), - 31 July 2013 (2013-07-31), pages 11-35, XP028734549, NEW YORK, NY, US ISSN: 0731-7085, DOI: 10.1016/J.JPBA.2013.07.013

## Description

### FIELD

The invention relates to the field of analytical chemistry, in particular to an impurity control method for cyclosporin A eye gel.

### BACKGROUND

Cyclosporine A (CyA) is a cyclic polypeptide consisting of 11 amino acids and is an active metabolite of a fungus in the soil. In 1978, CyA was first applied to clinical kidney transplantation in UK. After that, CyA was used for transplantation of liver, heart, lung, pancreas, bone marrow and other organs. All of them achieved satisfactory results and significantly improved the survival rate of patients. In 1984, cyclosporine A entered Chinese and formed a triple immunosuppressive regimen of "cyclosporine A + azathioprine + hormone", which greatly improved the survival rate of transplantation and also significantly reduced the incidence of acute rejection after transplantation. As a potent immunosuppressive agent, CyA has been researched more and more in recent years and is widely used in the treatment of various diseases.

Cyclosporine is poorly soluble. In order to making Cyclosporine into a uniform gel or liquid preparation, it is needed to be dissolved. Usually, cosolvents such as polyoxyethylene castor oil, castor oil, Tween and the like are added to ensure dissolution of cyclosporineand no precipitation during storage and use. However, the solubilizer generally has a strong UV peak, and there is a risk of excipient interference, and there are many process impurities and degradation impurities for cyclosporine. Existing impurities control methods for various raw materials cannot effectively detect these impurities.

Cyclosporine gel, cyclosporine ophthalmic emulsion and cyclosporine injection all contain solubilizers. These solubilizers have the following types: polyoxyethylene castor oil 35, polyoxyethylene castor oil 40, castor oil, etc.. These solubilizers have high UV response values, and strong peaks, and similar polarities to cyclosporine, so these excipients often have a significant impact on cyclosporine impurity detection. There is no patent publication on controlling impurities in cyclosporine preparations for the time being. There are no impurity control items and control methods in the literature and the cyclosporine preparations included in the pharmacopoeias of countries . F. Nunes Bonifacio et al., Journal of Pharmaceutical and Biomedical Analysis 49 (2009), 540-546 discloses the development and validation of a HPLC method for the determination of Cyclosporin A and its impurities in Neoral^{®} capsules and its generic versions

For ophthalmic preparations, the state requires management according to an injection, so the impurities control of the preparation is very strict. However, at present, in domestic and foreign patent documents and pharmacopoeias, no methods and limits for impurities are proposed for cyclosporine capsules, cyclosporine injections, etc., thus bringing risks to clinical applications and reducing the quality of products. How to solve the problem of excipient interference in the detection and the separation and control of many impurities has become an urgent problem to be solved.

The method that has been published for the control of impurities in cyclosporine is the method for cyclosporine drug substance in Chinese pharmacopoeia. The analytical methods are:
Chromatographic column: C₁₈ chromatographic column (150×4.6 mm, 5 µm)
The mobile phase: acetonitrile-water-methyl t-butyl ether-phosphoric acid (430: 520: 50: 1)
Flow rate: 1.0ml/min
Column temperature: 70 °C
Wave length: 220nm
Sample volume: 80 µl

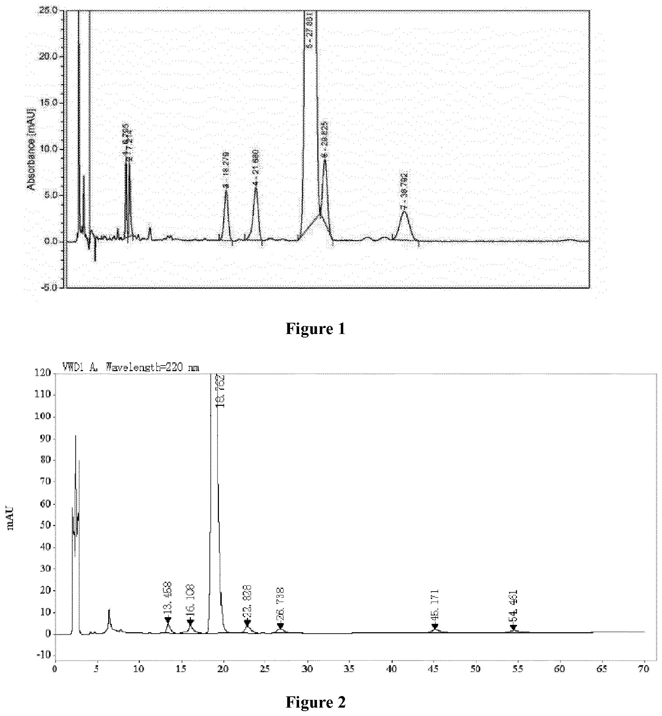

The problems of this method are: the specificity of detecting multiple impurities at the same time is not good, the separation degree of impurity from main peak is poor, the peaks of some impurities come out too early, and blank excipients interfere with detection of impurities having early peaks. The chromatogram of the mixed standard sample is shown in Figure1. As can be seen from Figure 1, the first two impurities are poorly separated, and baseline separation cannot be achieved for the impurities behind the main peak, and controls of multiple known impurities cannot be achieved simultaneously. The separation between impurities, impurity and main peak can not be achieved, the peak interference of excipients is serious, which affects the detection of impurity. Therefore, it is necessary to establish a new method for the detection of related substances, which can detect and isolate the above six known impurities (cyclosporine B, cyclosporin C, cyclosporine D, cyclosporin H, isocyclosporine A, isocyclosporine H) and unknown impurities.

### SUMMARY

The invention is set out in the appended set of claims.

An object of the present invention is to provide a method for controlling the impurities of cyclosporin A eye gel, which are determined by high performance liquid chromatography, wherein the chromatographic conditions are as follows:
The detection wavelength is 210-230 nm;
The column temperature is 60-68 °C;
The flow rate is 0.8-1 ml/min;
Mobile phase: THF-water-phosphoric acid.

Preferably, the column temperature is 65 °C.

The column is octadecylsilane-bonded silica gel as a filler (300 mm ^{∗} 3.9 mm, 4 um).

Preferably, the detection wavelength is 220 nm.

Preferably, the column is a waters column, a Thermo column, a Pheromone column or a YMC column.

The mobile phase has a volume ratio of THF-water-phosphoric acid of 400:600:1.58.

The method of the present invention adopts the chromatographic column octadecylsilane bonded silica gel as a filler (300 mm×3.9 mm, 4µm); the mobile phase is THF-water-phosphoric acid (400:600:1.58); the detection wavelength is 220 nm; the column temperature is 65°C, the flow rate is 0.8ml / min. The method can detect and isolate six known impurities cyclosporine B, cyclosporine C, cyclosporine D, cyclosporine H, isocyclosporine A, isocyclosporine H and unknown impurities, as shown in Figure 2. The limit for single known and unknown impurity is set at 1%. Animal experiments and clinical trials show that this method controls the preparation to achieve the standard preparation, showing good safety.

### BRIDF DESCRIPTION OF DRAWINGS

Figure 1 is an adaptive mixed standard chromatogram of the impurity system in Chinese Pharmacopoeia.
Figure 2 is an adaptive mixed standard chromatogram of the impurity system of example 12;
Figure 3 is an adaptive mixed standard chromatogram of the impurity system of example 1;
Figure 4 is an adaptive mixed standard chromatogram of the impurity system of example 2;
Figure 5 is an adaptive mixed standard chromatogram of the impurity system of example 3;
Figure 6 is an adaptive mixed standard chromatogram of the impurity system of example 4;
Figure 7 is an adaptive mixed standard chromatogram of the impurity system of example 5;
Figure 8 is an adaptive mixed standard chromatogram of the impurity system of example 6;
Figure 9 is an adaptive mixed standard chromatogram of the impurity system of example 7;
Figure 10 is an adaptive mixed standard chromatogram of an impurity system in example 8;
Figure 11 is an adaptive mixed standard chromatogram of the impurity system of example 9;
Figure 12 is an adaptive mixed standard chromatogram of the impurity system in example 10; and
Figure 13 is an adaptive mixed standard chromatogram of the impurity system in example 11.

### DETAILED DESCRIPTION

The present invention discloses a method for controlling impurities of cyclosporin A eye gel, and those skilled in the art can learn from the contents of the disclosure and appropriately improve the process parameters. It is to be understood that all such alternatives and modifications are apparent to those skilled in the art and are considered to be included in the present invention. The method of the present invention has been described in terms of preferred embodiments, and it is apparent that the method and application described herein may be changed or modified and combined to implement and apply the present invention without departing from the content, and scope of the invention.

In order to make those skilled in the art better understand the technical solutions of the present invention, the present invention will be further described in detail below with reference to specific examples.

### Example 1: chromatographic conditions:

Mobile phase: Acetonitrile-water-Methyl-t-butyl Ether-phosphoric acid (430: 520: 50: 1)
Wavelength: 210nm;
Flow rate: 1.0ml/min;
Column temperature: 70°C;
Sample volume: 80ul;
Chromatographic column: watersC18 (150mm^{∗}4.6mm, 5um) filler

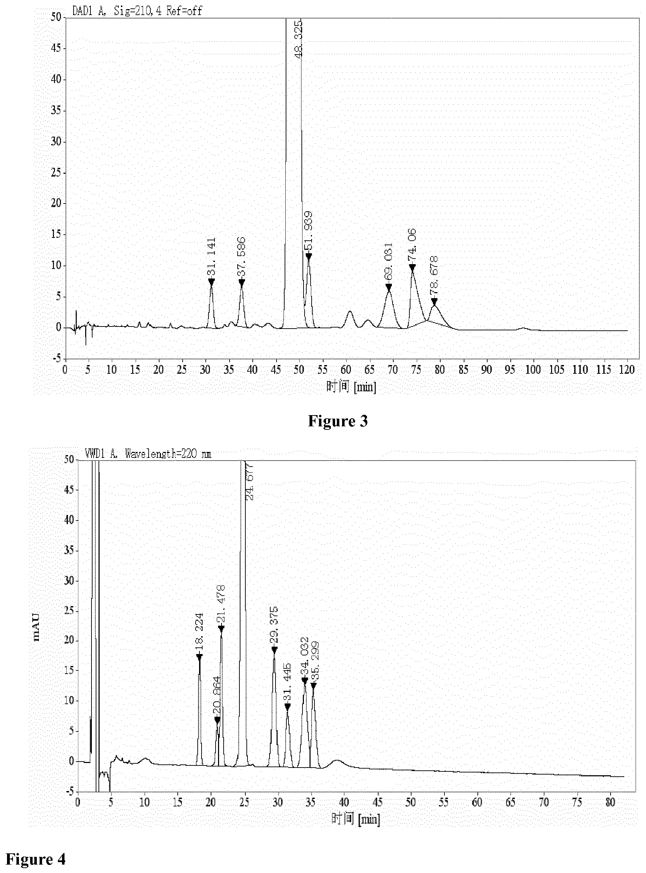

The solution chromatogram for the systematic application is shown in Figure 3. The results showed that there are many interference peaks interfering with the effective detection of impurities, and the separation degree of the main peak from the latter impurity (retention time 51.9 min) is poor, the last two impurities do not achieve baseline separation, therefore this method can not be used to detect the related substances of cyclosporine A eye gel.

### Example 2 chromatographic conditions:

Reference is made to article "Assay of Cyclosporin and its degradation products in cyclosporine capsules by HPLC method" of the Chinese Journal of antibiotics, Vol. 27, No. 4, April, 2002. The flow rate is 1 ml/min, HPLC cloume is HypersilBDSC18250mm^{∗}4.6mm,5um, Mobile phase: water-THF-0.4mol/L N-propylamine phosphate solution (0.4mol/L N-propylamine solution, adjusting pH value to 2.6 with phosphoric acid) = 590: 400: 10, column temperature is 70°C, and the wavelength is 220 nm.

The chromatogram is shown in Figure 4. The results showed that the separation degree of impurity was poor when the retention time was 34.0min or 35.3min, baseline separation was not achieved.

### Example 3 chromatographic conditions:

On the basis of example 2, the proportion of organic phases was reduced. The specific conditions are as follows: the flow rate is 1 ml/min, the HPLC column is Hypersil BDS C18 250mm^{∗}4.6mm, 5u, Mobile phase: [Water-0.4mol/L N-propylamine phosphate solution(0.4mol/L N-propylamine solution, adjusting pH value to 2.6 with phosphoric acid)=(885: 15)]-THF=650: 350, column temperature is 70°C, and the wavelength is 220 nm.

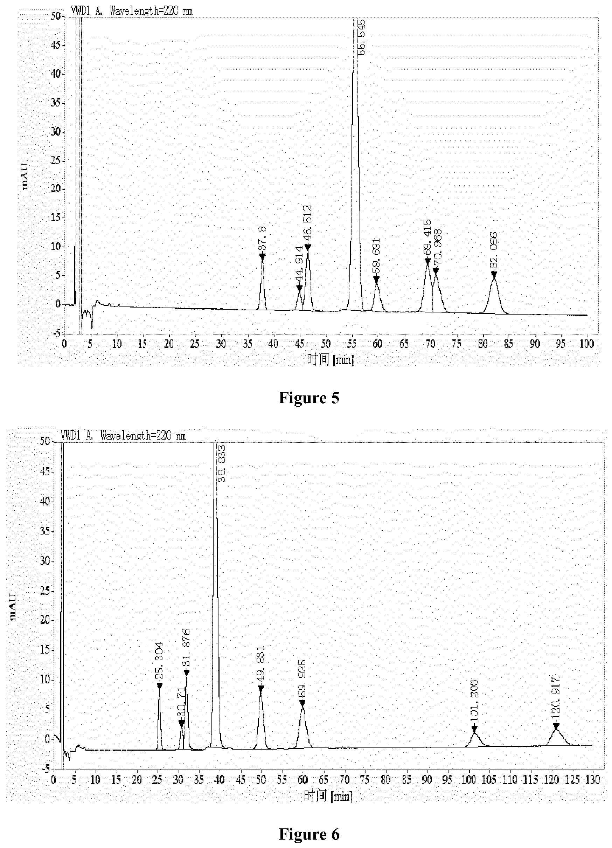

The chromatogram is shown in Figure 5. The results show that when the retention time was 69.4 min and 71.0 min, baseline separation of impurities was not achieved.

### Example 4 chromatographic conditions:

On the basis of example 3, the chromatographic column was changed. The specific conditions are as follows: the flow rate is 1ml/min, the HPLC column is Waters Nova-Pak C18 350mm^{∗}3.9mm, 4um, the mobile phase: [Water-0.4mol/L N-propylamine phosphate solution(0.4mol/L N-propylamine solution, adjusting pH value to 2.6 with phosphoric acid)=(885: 15)]-THF=650: 350, the column temperature is 70°C, and the wavelength is 220 nm.

The chromatogram is shown in figure 6. The result showed that the collecting time was too long , which was 130 minutes.

### Example 5 chromatographic conditions

On the basis of example 4, the isometric condition is changed to a gradient condition. Considering that the baseline noise of the mixed mobile phase through the instrument proportional valve is high, a mixed mobile phase was prepare. The specific conditions are as follows: the flow rate is 1ml/min, the HPLC column is Waters Nova-Pak C18 350mm^{∗}3.9mm, 4um, the mobile phase A: [Water-0.4mol/L N-propylamine phosphate solution(0.4mol/LN propylamine solution, adjusting pH value to 2.6 with phosphoric acid) = (885: 15)]-THF = 650: 370, the mobile phase B: [Water-0.4mol/L N-propylamine phosphate solution (0.4mol/LN propylamine solution, adjusting pH value to 2.6 with phosphoric acid)= (885: 15)]-THF = 600: 400, the column temperature is 70°C, and the wavelength is 220 nm.

**Gradient procedure:**

| Time (min) | A% | B% |
|---|---|---|
| 0 | 100 | 0 |
| 40 | 100 | 0 |
| 50 | 0 | 100 |
| 80 | 0 | 100 |
| 81 | 100 | 0 |
| \90 | 100 | 0, |

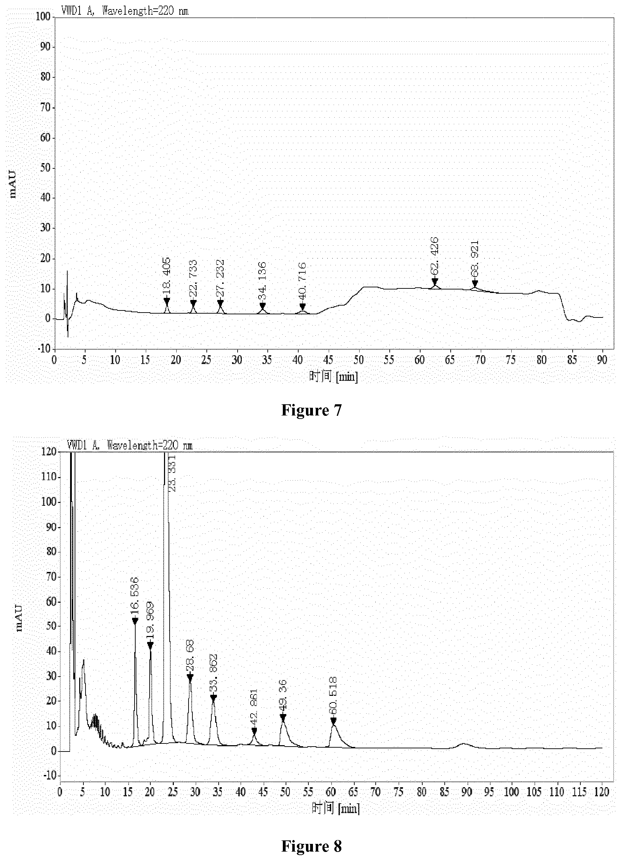

The chromatogram is shown in Figure 7. The results show that the baseline noise is large and impurity detection is interfered.

### Example 6 chromatographic conditions: It plans to remove N-propylamine from the mobile phase. The specific chromatographic conditions are as follows:

The flow rate is 0.7ml/min, the HPLC column is Waters Nova-Pak C18 350mm^{∗}3.9mm, 4um, the mobile phase: THF-water-85% phosphoric acid = 400: 600: 1.58 ml, the column temperature is 70°C, and the wavelength is 220nm.

The chromatogram is shown in Figure 8. The results showed that the acquisition time was 120 min and the acquisition time was too long.

### Example 7 chromatographic conditions:

It plans to change the amount of phosphoric acid in the mobile phase. The specific chromatographic conditions are as follows: the flow rate is 0.8ml/min, the HPLC column is Waters Nova-Pak C18 350mm^{∗}3.9mm, 4um, the mobile phase: THF- water-85% phosphoric acid = 400: 600: 1.8, the column temperature is 65°C, and the wavelength is 220nm.

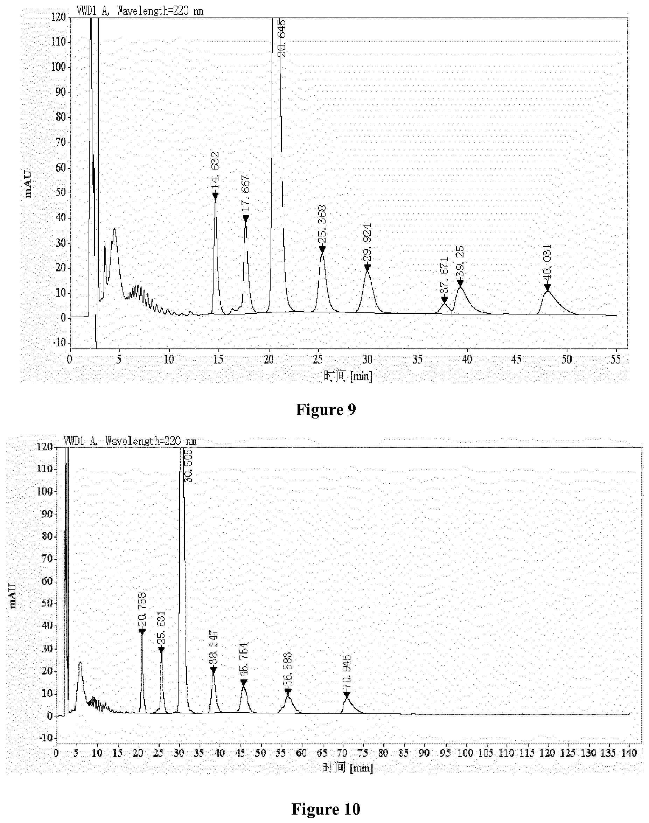

The chromatogram is shown in Figure 9. The results show that the detection of impurity at 39.3min was interfered by excipient peak at 37.7min.

### Example 8 chromatographic conditions:

It plan to reduce the proportion of organic phase in the mobile phase. The specific chromatographic conditions are as follows: the flow rate is 0.8ml/min, the HPLC column is Waters Nova-Pak C18 350mm^{∗}3.9mm, 4um, the mobile phase: THF-water-85% phosphoric acid= 380: 620: 1.58, the column temperature is 65°C, and the wavelength is 220nm.

The chromatogram is shown in Figure 10. The results showed that the peak of excipient at 140 min was not finished and the collecting time was too long.

### Example 9 chromatographic conditions:

Mobile phase: Water-tetrahydrofuran-85% phosphoric acid (600: 400: 1.58)
Wavelength: 220nm;
Flow rate: 0.7ml/min;
Column temperature: 70°C;
Sample volume: 80ul;
Chromatographic column: C18, 300mm^{∗}3.9mm, 4um

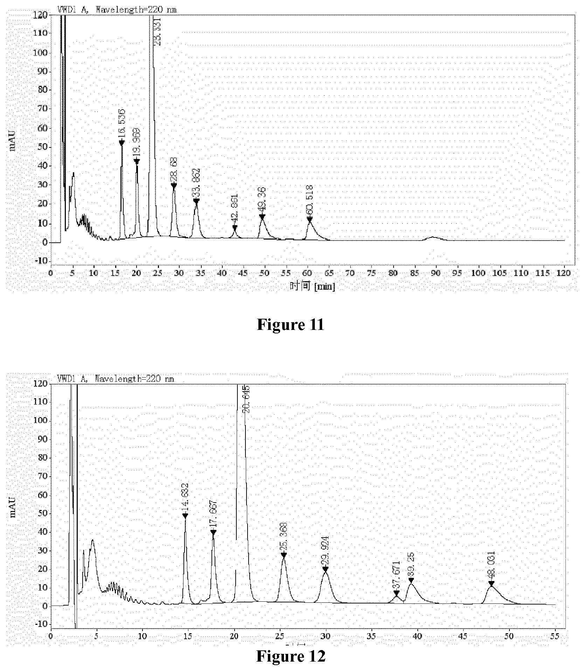

The chromatogram of the solution for systematic application is shown in Figure 11. What to be improved is: there is excipient peak at 90min and collecting time 120 minutes is too long.

### Example 10 chromatographic conditions:

Mobile phase: Water-tetrahydrofuran-85% phosphoric acid (600: 400: 1.80);
Wavelength: 220nm;
Flow rate: 0.8ml/min;
Column temperature: 65°C;
Chromatographic column: C18, 300mm^{∗}3.9mm, 4um
Sample volume: 100ul;

The chromatogram of the solution for systematic application is shown in Figure 12.

What to be improved is: the excipient peak 37 is not completely separated from the latter impurity and interferes with the impurity detection.

### Example 11 chromatographic conditions:

Mobile phase: Water-tetrahydrofuran-85% phosphoric acid (600: 400: 1.58);
Wavelength: 220nm;
Flow rate: 0.8ml/min;
Column temperature: 65°C;
Chromatographic column: C18, 300mm^{∗}3.9mm, 4um
Sample volume: 100ul;

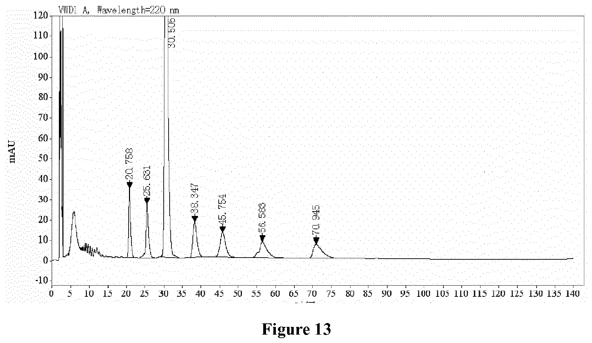

The chromatogram of the solution for systematic application is shown in Figure 13.

What to be improved is: the peak retention time of excipient was 140 minutes and the collecting time was too long.

### Example 12 chromatographic conditions:

Mobile phase: Water-tetrahydrofuran-85% phosphoric acid (600: 400: 1.58);
Wavelength: 220nm;
Flow rate: 0.8ml/min;
Column temperature: 65°C;
Chromatographic column: C18, 300mm^{∗}3.9mm, 4um
Sample volume: 100ul;

The chromatogram of the solution for systematic application is shown in Figure 2.

The above description only shows preferred embodiment of the present invention, and it should be noted that those skilled in the art can also make a number of improvements and modifications without departing from the principles of the present invention, and these improvements and modifications should be considered falling within the scope of protection of the present invention.

## Claims

1. A method of controlling the impurities in a cyclosporin A eye gel, which are determined by high performance liquid chromatography , wherein the chromatographic conditions are as follows:
the detection wavelength is 210-230 nm;
the flow rate is 0.8-1 ml/min;
the mobile phase is: THF-water-phosphoric acid, the chromatographic column has octadecylsilane-bonded silica gel as a filler, **characterized in that** the column temperature is 60-68° C; and the volume ratio of the mobile phase THF-water-phosphoric acid is 400:600:1.58.

2. The method of controlling impurities according to claim 1, **characterized in that** the column temperature is 65°C.

3. The method of controlling impurities according to claim 1, **characterized in that** the detection wavelength is 220 nm.

4. The method of controlling impurities according to claim 1, **characterized in that** the column is a Waters column, a Thermo column, a Pheromone column or a YMC column.

## Patentansprüche

1. Verfahren zum Bekämpfen der Verunreinigungen in einem Cyclosporin-A-Augengel, die durch Hochleistungsflüssigkeitschromatographie bestimmt werden, wobei die chromatographischen Bedingungen wie folgt sind:
die Detektionswellenlänge beträgt 210-230 nm;
die Durchflussrate beträgt 0,8-1 ml/min;
die mobile Phase ist: THF-Wasser-Phosphorsäure, die Chromatographiesäule weist als Füllstoff octadecylsilan-gebundenes Kieselgel auf, **dadurch gekennzeichnet, dass** die Säulentemperatur 60-68 °C beträgt;
und das Volumenverhältnis der mobilen Phase THF-Wasser-Phosphorsäure 400:600:1,8 beträgt.

2. Verfahren zum Bekämpfen von Verunreinigungen nach Anspruch 1, **dadurch gekennzeichnet, dass** die Säulentemperatur 65 °C beträgt.

3. Verfahren zum Bekämpfen von Verunreinigungen nach Anspruch 1, **dadurch gekennzeichnet, dass** die Detektionswellenlänge 220 nm beträgt.

4. Verfahren zum Bekämpfen von Verunreinigungen nach Anspruch 1, **dadurch gekennzeichnet, dass** die Säule eine Wassersäule, eine Thermosäule, eine Pheromonsäule oder eine YMC-Säule ist.

## Revendications

1. Procédé de contrôle des impuretés dans un gel oculaire à la cyclosporine A, qui sont déterminées par chromatographie liquide à haute performance, dans lequel les conditions chromatographiques sont les suivantes :
la longueur d'onde de détection est de 210 à 230 nm ;
le débit est de 0,8 à 1 ml/min ;
la phase mobile est : THF-eau-acide phosphorique, la colonne chromatographique contient comme charge du gel de silice lié à l'octadécylsilane, **caractérisé en ce que** la température de la colonne est de 60 à 68 °C ;
et le rapport volumique de la phase mobile THF-eau-acide phosphorique est de 400 :600 :1,8.

2. Procédé de contrôle des impuretés selon la revendication 1, **caractérisé en ce que** la température de la colonne est de 65 °C.

3. Procédé de contrôle des impuretés selon la revendication 1, **caractérisé en ce que** la longueur d'onde de détection est de 220 nm.

4. Procédé de contrôle des impuretés selon la revendication 1, **caractérisé en ce que** la colonne est une colonne de Waters, une colonne Thermo, une colonne Phéromone ou une colonne YMC.
